# EUROPÄISCHE PATENTANMELDUNG

(11) **EP 2 080 498 A1**
(43) Veröffentlichungstag der Anmeldung: **22.07.2009**
(21) Anmeldenummer: 08013055.2
(22) Anmeldetag: 18.07.2008
(51) Int. Cl.: A61G 5/10, A61G 5/12

(54) **Teilaktives Sitzorthesesystem und Herstellungsverfahren hierzu**

(30) Priorität: 20.07.2007 DE 102007034062
(71) Anmelder: Wagner mechanische Werkstätte für Orthopädietechnik, 90763 Fürth (DE)
(72) Erfinder: Wagner, Michael Harmut, 90763 Fürth (DE)

(57) **Zusammenfassung**

Die Erfindung beschreibt ein teilaktives Sitzorthesesystem bestehend aus mindestens einem Teilsystem (A,B,C) jeweils mit einer äußeren Stützschale (2) aus einem thermoplastischen Kunststoff und einer hiermit stoffschlüssig verbundenen inneren körpergerecht geformten Polsterschale (1) aus einem schaumartigen Werkstoff. Das Herstellungsverfahren hierzu besteht aus den Schritten: Erstellen einer Negativform durch Vakuumabdruck des Körpers des Patienten. Erstellen einer Positivform nach dem Stand der Technik;Temperaturbeaufschlagen des thermoplastischen schaumartigen Werkstoffs; Anformen des thermoplastischen schaumartigen Werkstoffs an die Positivform der Polsterschale; Temperaturbeaufschlagen des thermoplastischen Kunststoffs; Anformen des thermoplastischen Kunststoffs der Stützschale an und verbinden mit der Polsterschale.

## Beschreibung

Die Erfindung beschreibt ein teilaktives Sitzorthesesystem bevorzugt auf einem Rollstuhl befestigbar. Derartige Sitzorthesesysteme werden vorzugsweise bei Patienten eingesetzt, denen es auf Grund verschiedener Erkrankungen oder Insuffizienzen der knöcheren Struktur oder des Muskelapparates nicht möglich ist ohne Unterstützung für einen längeren Zeitraum zu sitzen.

Den Ausgangspunkt hierfür bilden bekannte Systeme gemäß der DE 195 20 585 C2 oder der DE 101 55 067 C2.

Die DE 195 20 585 C2 offenbart ein Sitzorthesesystem mit einer tragenden Mittelsäule, mit der ein Kopf- ein Sitz- und ein Unterschenkelrahmen verbunden sind. Zur Anpassung an die jeweilige Physiologie des Patienten dienen hierbei das anpassbare Sitzschalenmittelteil und die flexiblen Verbindungselemente zwischen den einzelnen Rahmen.

Das Sitzschalenmittelteil ist derart ausgebildet, dass es eine Mehrzahl von Pelotten aufweist. Diese sind in Längsrichtung als Thorax-, Becken- und Oberschenkelpelotten ausgebildet. Jede einzelne dieser Pelotten ist in sich spiegelbildlich ausgeführt und flexibel verstellbar. Durch diese Verstellbarkeit ist eine Anpassung an verschiedene Patienten möglich. Auf diesen Pelotten werden beispielhaft auswechselbare Polster und hierauf geeignete Wechselbezüge angeordnet.

Die DE 101 55 067 C2 offenbart eine speziell anpassbare Sitz- und Lehnenfläche insbesondere für Rollstühle. Hierbei weist die Sitz- und / oder Lehnenfläche mindestens ein mehrfach anformbares elastisches Stützpolster auf. Diese mehfache Anpassbarkeit wird mittels luftdichter, mehrfach evakuierbarer Kammern erreicht.

Ebenfalls bekannt ist die WO 96/12465 A1 welche ein Sitzorthesesystem offenbart, das aus einer Mehrzahl von Teilsystemen besteht, wobei die Sitzschale einen Boden aufweist, der mittels Verstelleinrichtungen an die Belange des Patienten anpassbar ist. Weiterhin bekannt ist die DE 20 2006 000 896 U1, die eine einteilige Sitzschale mit integrierte Rückenschale aus einem Kohlefaserwerkstoff offenbart.

Vorteilhaft bei beiden nach dem Stand der Technik bekannten Ausbildungen ist hierbei die Anpassbarkeit an verschiedene Patienten oder an verschiedene Wachstumstumsphasen. Nachteilig ist allerdings, dass die genannten Sitzorthesesysteme durch diese Anpassbarkeit sehr aufwendig und auch wenig pflege-, speziell reinigungsfreundlich sind.

Der Erfindung liegt die Aufgabe zugrunde ein Herstellungsverfahren für und ein hiermit hergestelltes teilaktives Sitzorthesesystem vorzustellen, das ein geringes Eigengewicht, die Möglichkeit zur flexiblen und einfachen Anpassung an den Patienten aufweist und diesem in definierten Grenzen ein dynamisches Sitzen ermöglicht.

Diese Aufgabe wird erfindungsgemäß gelöst durch ein Sitzorthesesystem mit den Merkmalen des Anspruchs 1 hergestellt mittels eines Verfahrens nach Anspruch 6. Bevorzugte Ausführungsformen sind in den Unteransprüchen beschrieben.

Ausgangspunkt des erfindungsgemäßen Sitzorthesesystem ist eine vorzugsweise aus einer Mehrzahl von Teilsystemen bestehende Anordnung. Jedes einzelne der Teilsysteme, die vorzugsweise ausgebildet sind als eine Sitzschale, eine Rückenschale und eine Kopfstütze, weist eine äußere Stützschale aus einem thermoplastischen Kunststoff auf. Mit dieser Stützschale ist eine Polsterschale stoffschlüssig verbunden. Diese Polsterschale selbst ist beispielhaft mittels des unten genannten Verfahrens körpergerecht geformt. Hierzu ist die Polsterschale aus einem schaumartigen Werkstoff ausgebildet.

Zur Ausbildung der teilaktiven Eigenschaft des Sitzorthesesystems weist der thermoplastische Kunststoff der Stützschale auch abhängig vom Gewicht des Patienten eine bevorzugte Dicke zwischen 6mm und 20mm auf. Die Polsterschale besitzt vorzugsweise eine maximale Dicke von 30mm.

Durch diese genannte Ausbildung der jeweiligen Teilsysteme ist es möglich, eine sehr gute Anpassung an die Körpermerkmale des Patient zu erreichen. Ebenso gestattet dieses Sitzorthesesystem eine nahezu ideale Unterstützung des Patienten dahingehend, dass einerseits eine Stützfunktion gegeben ist, während gleichzeitig durch die Beweglichkeit des Sitzorthesesystems dem Patienten ein gewisser Bewegungsspielraum in axialer, vertikaler und / oder horizontaler Richtung eröffnet wird. Durch diesen Bewegungsspielraum kann bei vorhandener Stützfunktion gleichzeitig eine muskelaufbauende oder erhaltende Funktion durch die Beweglichkeit der Teilsysteme erreicht werden.

Weiterhin erweist sich der erfindungsgemäße Aufbau des Sitzorthesesystem als leichtgewichtig und einfach zu reinigen. In Verbindung mit einem geeigneten Überzug aus einem waschbaren und atmungsaktiven Stoff ergibt sich ein äußerst vorteilhaftes Sitzorthesesystem auch für den Einsatz bei inkontinenten Patienten.

Es ist weiterhin bevorzugt, wenn die drei Teilsysteme mittels metallischer Verbindungseinrichtungen zueinander justierbar angeordnet sind. Hierdurch ist eine optimale Anpassung der Sitzschale, der Rückenschale und der Kopfstütze zueinander und in geeigneter Weise an die Physiologie des Patienten möglich.

Diese metallischen Verbindungseinrichtungen werden gebildet durch eine erfindungsgemäße Gesamtverbindungseinrichtung, die aus zwei parallel angeordneten und mittels Querverbindungseinrichtungen verbundenen L-förmigen Grundkörpern besteht. Zwischen dem jeweils ersten und dem zweiten Schenkel des jeweiligen Grundkörpers ist hierbei ein feststellbares Drehgelenk angeordnet. Weiterhin ist mindestens eine weitere dieser Querverbindungseinrichungen entlang der beiden zweiten Schenkels verschieb- und fixierbar angeordnet ist. Zur Fixierung dienen hier bekannte und übliche Klemmeinrichtungen.

Das erfindungsgemäße Sitzorthesesystem ist durch diesen Aufbau und seine teilaktive Ausgestaltung besonders geeignet, um mittels der ersten Schenkel auf einem Rollstuhl montiert zu werden wozu mindestens drei Befestigungseinrichtungen an den beiden ersten Schenkeln der Verbindungseinrichtung und einer dort angeordneten Querverbindungseinrichung vorzusehen sind.

Das erfindungsgemäße Verfahren zur Herstellung eines derartigen Sitzorthesesystem weist folgende wesentliche Schritte auf:
• Erstellen einer Negativform durch Vakuumabdruck des Körpers des Patienten.
• Erstellen einer Positivform pro Teilsystem, vorzugsweise ausgebildet als ein Gipspositiv, nach dem Stand der Technik.
• Weiterbilden der Positivform zu späteren Anordnung eines zusätzlichen Polster und / oder eines Bezugstoffes.
• Temperaturbeaufschlagen des vorzugsweise in Plattenform ausgebildeten thermoplastische schaumartigen Werkstoffs.
• Anformen, vorzugsweise mittels Vakuum, des thermoplastischen schaumaritigen Werkstoffs an die Positivform zur Ausbildung der Polsterschale.
• Temperaturbeaufschlagen des vorzugsweise in Plattenform ausgebildeten thermoplastische Kunststoffs.
• Anformen, vorzugsweise mittels Vakuum, des thermoplastischen Kunststoffs der Stützschale an und stoffschlüssiges verbinden, vorzugsweise mittels kleben, mit der Polsterschale.
• Nachbearbeiten der äußeren Kanten der jeweiligen Teilsysteme.
• Anordnen und verbinden der Teilsystem mittels metallischer Verbindungseinrichtungen.

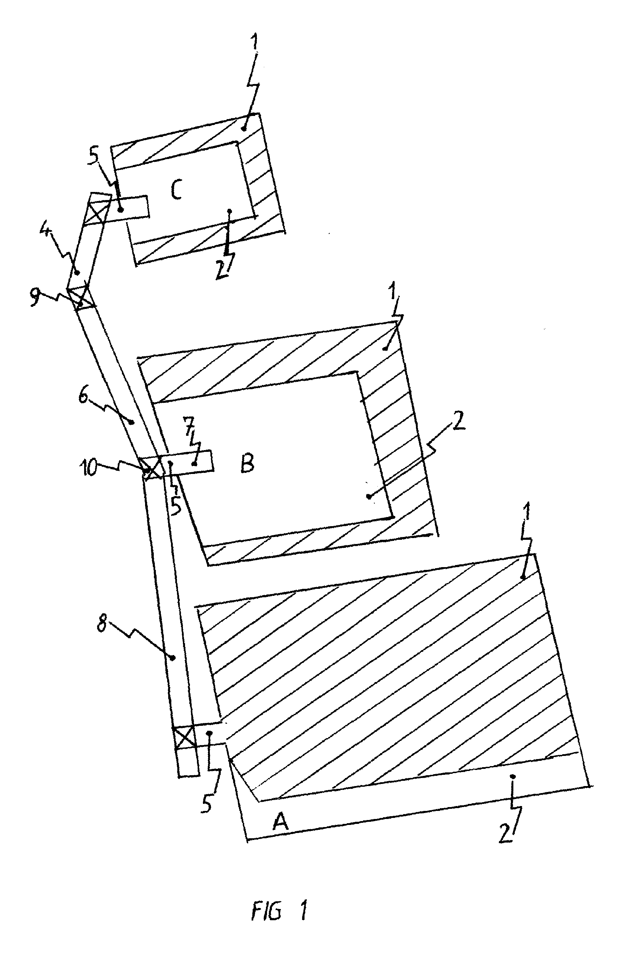

Besonders bevorzugte Weiterbildungen dieses Sitzorthesesystem sind in der Beschreibung der folgenden Ausführungsbeispiele genannt. Die erfinderische Lösung wird zudem an Hand der Ausführungsbeispiele gemäß den Fig. 1 bis 3 weiter erläutert.

Fig. 1 zeigt ein das Sitzorthesesystem gemäß DE 10 2007 034 062 A1 bestehend aus einer Sitzschale (A), einer Rückenschale (B) und einer Kopfstütze (C). Diese Teilsysteme (A, B, C) sind mittels metallischer Verbindungseinrichtungen zueinander justierbar angeordnet. Hierzu ist der Kopfstütze (C) eine erste Verbindungseinrichtung (4) zugeordnet, die mittels einer Halteeinrichtung (5) mit der Kopfstütze verbunden ist.

Ebenso ist der Rückenschale (B) eine zweite Verbindungseinrichtung (6) zugeordnet, die mittels einer Halteeinrichtung (7) mit der Rückenschale (B) verbunden ist. In gleicher Weise weist die Sitzschale (A) eine zugeordnete dritte Verbindungseinrichtung (8) und eine nicht dargestellte Halteeinrichtung auf.

Die Verbindungseinrichtungen (4, 6, 8) sind mittels Justageelementen (9, 10) derart miteinander verbunden, dass die Teilsysteme des erfindungsgemäßen Sitzorthesesystems patientengerecht zueinander anordenbar sind.

Hierzu sind die Justageelemente (9, 10) vorzugsweise mittels nicht dargestellter Feststelleinrichtungen ausgebildet und somit einfach einstell- bzw. veränderbar.

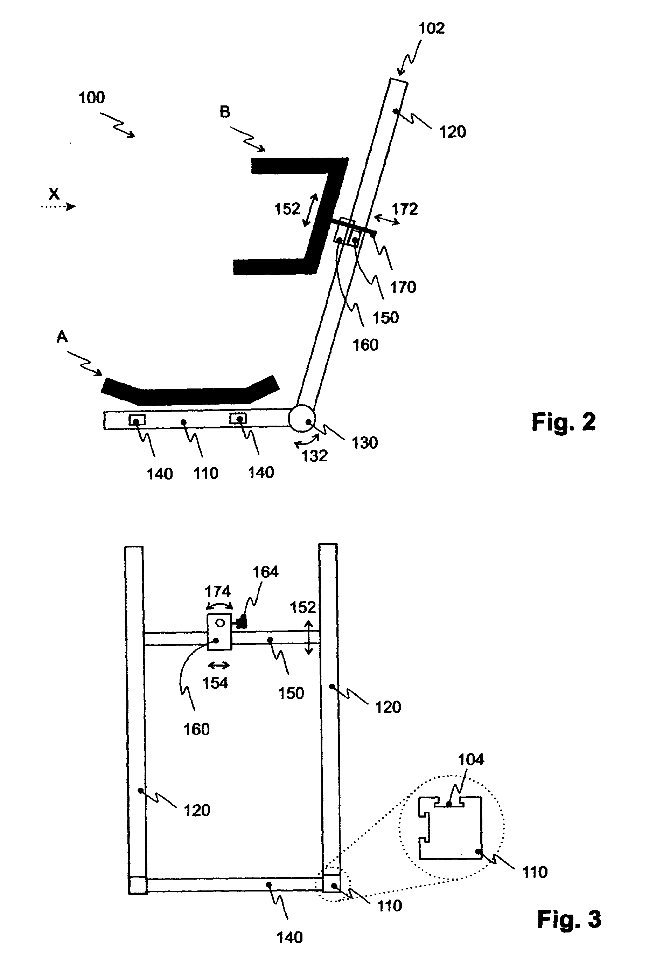

Fig. 2 zeigt in Seitenansicht eine Weiterbildung der Verbindungseinrichtungen (4, 6, 8) gemäß Fig. 1 zu einer Gesamtverbindungseinrichtung (100), während Fig. 3 eine Frontansicht aus Richtung (X, vgl. Fig. 2) zeigt. Die Gesamtverbindungseinrichtung (100) dient der Anordnung der drei schematisch angedeuteten Teilsysteme (A, B, C) einer Sitzschale (A), einer Rückenschale (B) und nicht explizit dargestellt einer Kopfstütze (C, vgl. Fig. 1) in möglichst flexibler weise zueinander. Hierzu ist die Gesamtverbindungseinrichtung (100) ausgebildet mit zwei parallel angeordneten L-förmigen Grundkörpern (102).

Jeder diese beiden Grundkörper (102) weist jeweils einen ersten (110) und zweiten (120) Schenkel auf. Auf den beiden ersten Schenkeln (110) ist die Sitzschale (A) angeordnet. Hierzu sind die beiden ersten Schenkel (110) mittels mindestens zwei Querverbindungseinrichtungen (140) miteinander starr verbundenen. Mittels eines feststellbaren und somit einfach und flexibel auf die Bedürfnisse des Patienten einzustellenden Drehgelenks (130) ist der jeweilige ersten (110) mit dem jeweiligen zweiten (120) Schenkel verbunden.

Diese zweiten Schenkel (120) sind mit mindestens einer, vorzugsweise allerdings mit zwei weiteren Querverbindungseinrichtungen (150) miteinander verbunden. Die Verbindung der zweiten Schenkel (120) mit diesen beiden Querverbindungseinrichtungen (150) sind derart ausgebildet, dass die Querverbindungseinrichtungen (150) entlang der zweiten Schenkel (120) verschiebbar (152) und fixierbar sind. Hierzu weisen die zweiten Schenkel (120) einen quadratischen, möglicherweise aber auch einen rechteckigen, runden oder ovalen Querschnitt und und in ihrer Längsrichtung vorteilhafterweise mindestens eine Nut (104) auf. In dieser Nut (104) ist eine Befestigungseinrichtung, vorzugsweise eine Schraubverbindung, für die Querverbindungseinrichtung (150) der zweiten Schenkel (120) verstellbar fixiert, wodurch eine Höhenanpassung (152) einer hiermit verbundenen Rückenschale (B) oder einer nicht dargestellten Kopfstütze (C, vgl. Fig. 1) möglich ist.

Weiterhin weist diese weitere Querverbindungseinrichtung (150) der zweiten Schenkel (120) eine Aufnahmeeinrichtung (160) für ein vorzugsweise stabförmiges Halteelement (170) auf. Dieses Halteelement (170) ist vorzugsweise starr mit der Rückenschale (B) oder der Kopfstütze (C, vgl. Fig. 1) verbunden und ragt im wesentlichen senkrecht von dieser in Richtung der Querverbindungseinrichtung (150) weg.

Die Aufnahmeeinrichtung (160) weist eine Durchführung (162) mit Klemmeinrichtung (164) für dieses, vorzugsweise mit rundem Querschnitt ausgebildete, Halteelement (170) auf, wodurch die hiermit verbundene Rückenschale (B) oder Kopfstütze (C, vgl. Fig. 1) zu der Querverbindungseinrichtung (150) der zweiten Schenkel (120) flexibel angeordnet werden kann. Dieses Anordnung erlaubt eine Verstellbarkeit in Längsrichtung (172) des Halteelements (170) wie auch eine Verstellbarkeit (174) der Rückenschale (B) oder der Kopfstütze (C, vgl. Fig. 1) um die Längsachse des Halteelements (170) als Drehachse.

Mittels bekannter Klemmeinrichtung wird einerseits die Verstellbarkeit und andererseits die Fixierbarkeit und damit Stabilität nach erfolgter Einstellung auf die jeweiligen Ansprüche des Patienten gewährleistet.

Weiterhin kann es vorteilhaft sein die jeweilige Querverbindungseinrichtung (150) der beiden zweiten Schenkel (120) derart auszubilden, dass die Aufnahmeeinrichtung (160) längs dieser Querverbindungseinrichung (150) verschiebbar und selbstverständlich auch fixierbar ist. Hierzu ist es bevorzugt auch diese Querverbindungseinrichtung (150) analog der Grundkörper (102) auszubilden und eine Nut (104) vorzusehen um diese Verstellbarkeit zu gewährleisten.

## Patentansprüche

1. Teilaktives Sitzorthesesystem bestehend aus mindestens einem Teilsystem (A, B, C) jeweils mit einer äußeren Stützschale (2) aus einem thermoplastischen Kunststoff und einer hiermit stoffschlüssig verbundenen inneren körpergerecht geformten Polsterschale (1) aus einem schaumartigen Werkstoff.

2. Sitzorthesesystem nach Anspruch 1,
bestehend aus den drei Teilsystemen Sitzschale (A), Rückenschale (B) und Kopfstütze (C).

3. Sitzorthesesystem nach Anspruch 2,
wobei die drei Teilsysteme (A, B, C) mittels metallischer Verbindungseinrichtungen (4 bis 10) zueinander justierbar angeordnet sind.

4. Sitzorthesesystem nach Anspruch 1,
wobei dieses auf einem Rollstuhl montierbar ausgebildet ist und hierzu mindestens drei Befestigungseinrichtungen aufweist.

5. Sitzorthesesystem nach Anspruch 1,
wobei der thermoplastische Kunststoff der Stützschale (2) eine Dicke zwischen 6mm und 20mm aufweist und wobei die Polsterschale (1) eine maximale Dicke von 30mm aufweist.

6. Sitzorthesesystem nach Anspruch 3,
wobei metallischen Verbindungseinrichtungen eine Gesamtverbindungseinrichtung (100) bilden, die aus zwei parallel angeordneten und mittels Querverbindungseinrichtungen (140, 150) verbundenen L-förmigen Grundkörpern (102) besteht, wobei zwischen dem jeweils ersten (110) und zweiten (120) Schenkel der Grundkörper (102) ein feststellbares Drehgelenk (130) angeordnet ist und mindestens eine weitere Querverbindungseinrichtung (150) entlang der zweiten Schenkel (120) verschieb- und fixierbar ausgebildet ist.

7. Sitzorthesesystem nach Anspruch 1,
wobei diese mindestens eine weitere Querverbindungseinrichtung (150) der zweiten Schenkel (120) jeweils eine Aufnahmeeinrichtung (160) für ein an einem Teilsystem (A, B, C) befestigtes stabförmiges Halteelement (170) aufweist und dieses Halteelement (170) in der Aufnahmeeinrichtung (160) entlang seiner Längsachse verschieb- (172) und fixierbar und/oder um seine Längsachse dreh- (174) und fixierbar ausgebildet ist.

8. Sitzorthesesystem nach Anspruch 1,
wobei der jeweilige Grundkörper (102) und die Querverbindungseinrichtungen (140, 150) einen quadratischen, rechteckigen, runden oder ovalen Querschnitt aufweisen und in ihrer Längsrichtung mindestens eine Nut (104) aufweisen.

9. Verfahren zur Herstellung eines Sitzorthesesystem nach einem der vorhergehenden Ansprüche, mit den Herstellungsschritten:
• Erstellen einer Negativform durch Vakuumabdruck des Körpers des Patienten.
• Erstellen einer Positivform nach dem Stand der Technik.
• Temperaturbeaufschlagen des thermoplastische schaumartigen Werkstoffs.
• Anformen des thermoplastischen schaumaritigen Werkstoffs an die Positivform der Polsterschale (1).
• Temperaturbeaufschlagen thermoplastische Kunststoffs.
• Anformen des thermoplastischen Kunststoffs der Stützschale (2) an und verbinden mit der Polsterschale (1).

10. Verfahren nach Anspruch 9,
wobei im Anschluss die Teilsysteme (A, B, C) zu einen Sitzorthesesystem angeordnet und mittels metallischer Verbindungseinrichtungen (4 bis 10) verbundenen werden.
